# DEMANDE DE BREVET EUROPEEN

(11) **EP 1 005 836 A1**
(43) Date de publication de la demande: **07.06.2000**
(21) Numéro de dépôt: 98122951.1
(22) Date de dépôt: 03.12.1998
(51) Int. Cl.: A61B 10/00, A61B 17/28

(54) **Instrument chirurgical comprenant une unité de commande et une unité fonctionelle séparables**

(71) Demandeur: Nivarox-FAR S.A., CH-2400 Le Locle (CH)
(72) Inventeur: Peters, Jean-Bernard, 2300 La Chaux-de-Fonds (CH); Vorpe, Sacha, 2605 Sonceboz (CH)
(74) Mandataire: Ravenel, Thierry Gérard Louis

(57) **Abrégé**

L'invention concerne un instrument chirurgical comprenant: une unité fonctionnelle et une unité de commande pouvant être séparée de celle-ci, l'unité fonctionnelle comprenant une gaine ayant une extrémité proximale et une extrémité distale, un outil monté à l'extrémité distale de la gaine, et un câble coulissant à l'intérieur de la gaine reliant ledit outil à l'unité de commande, et permettant d'actionner l'outil lorsque l'on agit sur ledit câble au moyen de l'unité de commande,
- l'unité de commande comprenant des premiers et des deuxièmes moyens de couplage pour relier, de façon amovible, l'unité de commande respectivement à l'extrémité proximale de la gaine et à l'extrémité proximale du câble d'instrument étant caractérisé en ce que l'unité de commande comprend des premiers et deuxièmes moyens d'actionnement, et en ce que les premiers moyens d'actionnement agissent simultanément sur les premiers et deuxièmes moyens de couplage pour saisir ou libérer ladite gaine et ledit câble tandis que les deuxièmes moyens d'actionnement n'agissent que sur les deuxièmes moyens de couplage pour commander la translation relative de la gaine et du câble.

## Description

La présente invention concerne un instrument chirurgical comprenant une unité de commande et une unité fonctionnelle séparables. Plus particulièrement l'invention concerne un tel instrument comprenant une unité fonctionnelle formée d'un câble de commande coulissant à l'intérieur d'une gaine souple et relié à un outil, tel qu'une pince à biopsie, commandé à distance par une unité de commande pouvant être séparée aisément de l'unité fonctionnelle.

Les pinces à biopsie sont notamment utilisées pour effectuer chez un patient des prélèvements de tissus sur des organes vivants, par exemple en vue d'examens cliniques. Ces pinces comprennent classiquement des mâchoires coupantes actionnées à distance par une poignée de commande reliée aux mâchoires par un système de câble coulissant dans une gaine souple. Une pince à biopsie de ce type est décrite dans le brevet US 5,582,617. La pince et sa gaine souple sont introduites dans le tube ou canule d'un endoscope par l'intermédiaire duquel la pince est amenée dans le corps du patient à l'emplacement de prélèvement souhaité, une caméra ou une lentille grossissante est également introduite dans ce tube ou un autre tube pour que le chirurgien puisse contrôler l'opération.

Pendant longtemps, ces instruments étaient très chers en raison de la très petite taille des mécanismes, des matériaux et des procédés de fabrication qu'ils mettent en oeuvre. Il est toutefois apparu ces dernières années sur le marché des instruments à usage unique. Ce nouveau type d'instruments permet notamment d'éliminer les problèmes liés à la stérilisation des instruments à usages multiples et d'éliminer ainsi d'éventuels risques de contamination croisée entre patients. Ces instruments à usage unique comprennent généralement une poignée de commande, qui est réutilisable, associée de façon amovible à une unité fonctionnelle jetable. Un exemple d'un instrument de ce type est décrit dans le brevet US 4,763,668. Ce document décrit en particulier une pince à biopsie comprenant une poignée de commande reliée de façon amovible à l'unité fonctionnelle. Plus précisément les moyens de liaison comprennent une pluralité de vis qui fixent par coincement le câble de commande des mâchoires dans une partie de la poignée ainsi qu'une douille filetée dans laquelle l'extrémité proximale de la gaine est vissée. La présence de ces vis nécessite l'utilisation d'outils, ce qui complique les opérations de fixation et de désolidarisation de la poignée avec l'unité fonctionnelle.

La présente invention a donc pour but principal de remédier aux inconvénients de l'art antérieur susmentionné, en fournissant un instrument chirurgical comprenant une unité fonctionnelle et une unité de commande qui peuvent être facilement désolidarisées l'une de l'autre et assemblées l'une à l'autre de manière simple, fiable et rapide, et notamment sans avoir à utiliser d'outils particuliers.

L'invention a également pour but de fournir un instrument chirurgical comprenant une unité fonctionnelle et une unité de commande qui peuvent être désolidarisées l'une de l'autre à l'aide d'une seule main.

L'invention a également pour but de fournir un instrument chirurgical du type défini ci-dessus ayant une unité de commande compacte qui offre également une grande précision de manipulation ainsi qu'une utilisation aisée.

A cet effet, l'invention a pour objet un instrument chirurgical comprenant:
- une unité fonctionnelle et une unité de commande pouvant être séparée de celle-ci, l'unité fonctionnelle comprenant une gaine ayant une extrémité proximale et une extrémité distale, un outil monté à l'extrémité distale de la gaine, et un câble coulissant à l'intérieur de la gaine reliant ledit outil à l'unité de commande, et permettant d'actionner l'outil lorsque l'on agit sur ledit câble au moyen de l'unité de commande,
- l'unité de commande comprenant des premiers et des deuxièmes moyens de couplage pour relier, de façon amovible, l'unité de commande respectivement à l'extrémité proximale de la gaine et à l'extrémité proximale du câble caractérisé en ce que
   l'unité de commande comprend des premiers et deuxièmes moyens d'actionnement, et en ce que les premiers moyens d'actionnement agissent simultanément sur les premiers et deuxièmes moyens de couplage pour saisir ou libérer ladite gaine et ledit câble tandis que les deuxièmes moyens d'actionnement n'agissent que sur les deuxièmes moyens de couplage pour commander la translation relative de la gaine et du câble.

Un avantage de l'instrument chirurgical selon l'invention réside dans le fait que la gaine et le câble de l'unité fonctionnelle peuvent être attachés à l'unité de commande respectivement détachés de celle-ci, de façon simultanée et en n'utilisant aucun outil. Un autre avantage de l'instrument chirurgical selon l'invention réside dans le fait qu'il permet l'utilisation de gaines jetables, l'unité de commande étant à usages multiples.

Selon un mode réalisation avantageux de l'invention, l'unité de commande comprend en outre des moyens d'éjection de l'unité fonctionnelle, les moyens d'éjection comprenant un poussoir qui coulisse dans la partie distale de l'unité de commande, et qui est associé à des moyens de rappel agencés pour que le poussoir pousse l'extrémité proximale de l'unité fonctionnelle vers l'extérieur de l'unité de commande.

Grâce à ces moyens d'éjection, l'opération de désolidarisation peut être réalisée à l'aide d'une seule main.

Selon un autre mode de réalisation avantageux de l'invention, l'unité de commande comprend en outre des moyens de connexion électriques permettant de porter les deuxièmes moyens de couplage à un potentiel déterminé.

Ainsi, l'instrument peut réaliser, le cas échéant, des opérations de cautérisation par exemple à l'aide d'une anse à politectomie reliée à l'extrémité du câble en faisant passer du courant à haute fréquence à travers la anse.

D'autres caractéristiques et avantages de la présente invention apparaîtront dans la description suivante d'un mode de réalisation préféré, présenté à titre d'exemple non limitatif en référence aux dessins annexés, dans lesquels:
- la figure 1 est une coupe longitudinale schématique partielle de l'instrument chirurgical selon l'invention, la gaine et le câble étant attachés à l'unité de commande;
- la figure 2 est une coupe longitudinale schématique partielle de l'unité de commande dans une position dans laquelle la gaine et le câble peuvent être attachés à l'unité de commande respectivement détachés de celle-ci;
- la figure 3 est une vue éclatée d'un mode de réalisation de l'unité de commande de l'instrument chirurgical selon l'invention
- la figure 4a est une coupe selon la ligne II-II de la figure 2;
- la figure 4b montre un détail de l'unité de commande;
- la figure 5a est une coupe partielle longitudinale de la partie distale de l'unité de commande illustrant une variante de réalisation comprenant des moyens d'éjection de l'unité fonctionnelle;
- la figure 5b est une vue en perspective de la partie distale représentée à la figure 5a, et
- la figure 6 est une coupe partielle longitudinale de la partie proximale de l'unité de commande illustrant une variante de réalisation comprenant des moyens de connexion électriques permettant de porter les deuxièmes moyens de couplage à un potentiel déterminé.

L'instrument chirurgical selon l'invention va être décrit dans son application au prélèvement de tissus sur des organes vivants ou biopsie. Il va de soi néanmoins que cet instrument peut être utilisé à d'autres fins pour la préhension ou le prélèvement d'échantillons de fragments ou de particules de natures diverses organiques, minérales ou autres.

En se référant aux figures on voit un instrument chirurgical selon l'invention désigné par la référence numérique générale 1. Cet instrument 1 comprend une unité fonctionnelle 2 et une unité de commande 4 pouvant être séparée de celle-ci.

L'unité fonctionnelle 2 comprend une gaine 6 souple ayant une extrémité proximale 8 et une extrémité distale 10 et un outil 12, en l'occurrence une pince à deux mâchoires pivotantes, montée à l'extrémité distale 10. La gaine 6 est par exemple formée d'un ressort hélicoïdal à spires jointives. Un câble 14 coulisse dans la gaine 6 et relie la pince 12 à l'unité de commande 4. L'actionnement de l'unité de commande 4 permet de faire coulisser le câble 14 relativement à la gaine 6 et ainsi de commander l'ouverture et la fermeture des mâchoires de la pince 12.

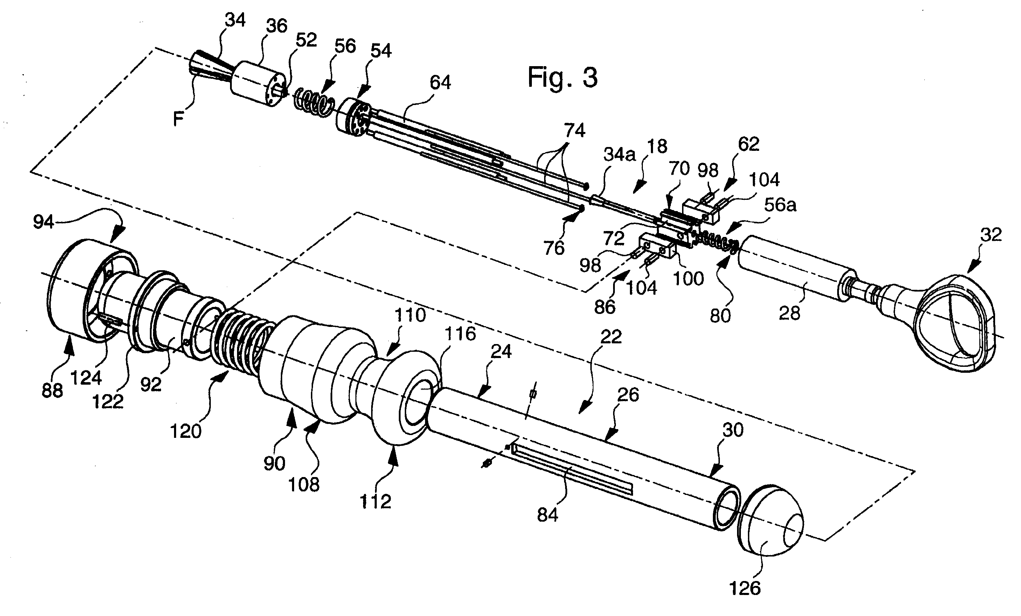

L'unité de commande 4 comprend des premiers moyens de couplage 16 (figure 3) pour relier de façon amovible l'unité de commande 4 à l'extrémité proximale 8 de la gaine 6. L'unité de commande 4 comprend également des deuxièmes moyens de couplage 18, pour relier l'unité de commande à l'extrémité proximale 20 du câble 14.

L'unité de commande 4 comprend un corps allongé creux 22 qui présente, dans l'exemple décrit, une forme cylindrique. Les moyens de couplage 16 sont disposés dans une région d'extrémité avant 24 du corps 22, destinée à recevoir les extrémités proximales 8 et 20 respectivement de la gaine 6 et du câble 14 tandis que les moyens de couplage 18 sont disposés dans une région médiane 26 du corps 22 destinée à recevoir l'extrémité proximale 20 du câble 14 (figure 3). Un bouchon arrière 28 est logé dans une région d'extrémité arrière 30 du corps 22 et une poignée de manipulation 32 est fixée au bouchon arrière 28 et s'étend à l'extérieur du corps 22 depuis la partie arrière du bouchon 28.

Les moyens de couplage 16 sont montés fixement par rapport au corps 22 et comprennent un dispositif à pince. Dans l'exemple représenté, le dispositif à pince comprend une pince 34 dont l'ouverture et la fermeture est commandé par un manchon de commande conique 36. Plus précisément, la pince 34 présente la forme d'une pince américaine (chuck) comportant une partie avant 38 conique prolongée par une partie arrière cylindrique 40 traversées toutes les deux longitudinalement par un alésage central 42 débouchant. La pince 34 comporte en outre dans sa partie avant 38 et sur une portion de sa partie arrière plusieurs fentes longitudinales F décalées angulairement les unes par rapport aux autres. Ces fentes définissent ainsi des mâchoires permettant de saisir l'extrémité proximale 8 de la gaine 6. Dans l'exemple décrit les fentes et les mâchoires sont au nombre de quatre. L'alésage central 42 comprend une partie avant 44 prolongée par une partie arrière 46 ayant un diamètre inférieur à celui de la partie avant 44. Le diamètre de la partie avant 44 est prévu pour recevoir et pincer l'extrémité proximale 8 de la gaine 6 tandis que le diamètre de la partie arrière 46 est prévu pour être traversé par le câble 14, ce dernier pouvant y coulisser librement. On notera que la partie 44 comprend une rainure 48 destinée à recevoir un bourrelet 50 prévu à l'extrémité de la gaine 8, augmentant ainsi la sécurité d'accrochage de la gaine 6 dans la pince 34.

L'extrémité 52 de la partie arrière 40 comporte un filetage et est vissée dans un bouchon avant 54 monté fixe dans le corps 22. Le manchon conique 36 comprend une surface extérieure cylindrique ajustée à coulissement à l'intérieur du corps 22 et un alésage intérieur ayant une conicité complémentaire à celle de la partie avant 38 (figure 3). Des moyens de rappel 56, formés par un ressort hélicoïdal, s'étendent autour de la partie arrière 40 de la pince 34 entre une surface de butée 58 du bouchon avant 54 et la face frontale arrière 60 du manchon conique 36. Le ressort 56 est arrangé de manière à solliciter le manchon conique vers l'avant du corps afin de fermer les mâchoires de la pince 34.

Les moyens de couplage 18 sont montés à coulissement par rapport au corps 22 et comprennent également un dispositif à pince du type de celui qui vient d'être décrit en liaison avec les moyens de couplage 16. Comme la structure des moyens de couplage 18 est sensiblement la même que celle des moyens de couplage 16, les mêmes éléments portent les mêmes références complétées par la lettre "a" pour les moyens de couplage 18.

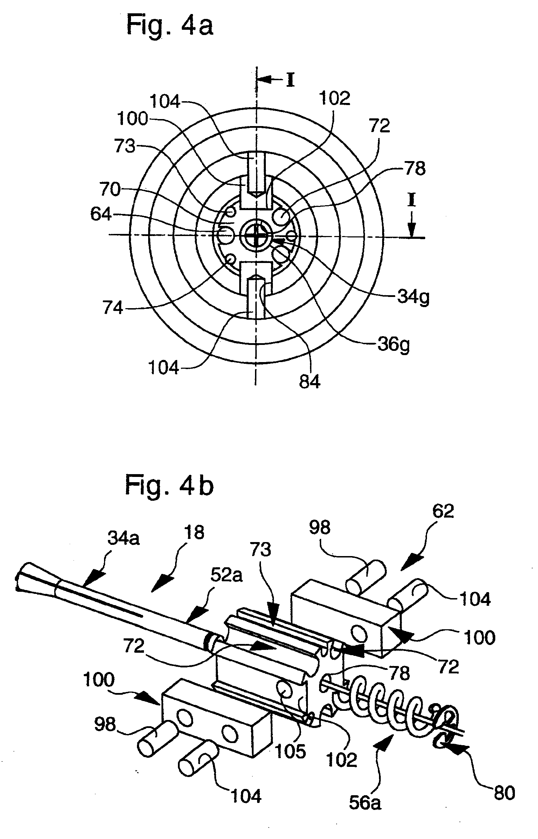

La pince 34a est associée à un équipage mobile 62 qui coulisse dans le corps 22 le long d'entretoises 64 qui s'étendent parallèlement à l'axe longitudinal du corps 22 entre la face arrière 66 du bouchon avant 54 et la face avant 68 du bouchon arrière 28. Dans l'exemple décrit, l'équipage mobile 62 comprend un coulisseau 70 ayant une forme généralement cylindrique et comprenant sur sa surface extérieure trois rainures longitudinales 72 (figures 3 et 4b) recevant les entretoises 64. Le coulisseau 70 comprend en outre sur sa surface extérieure trois autres rainures longitudinales 73 dans lesquelles coulissent respectivement des tirants 74 qui s'étendent à travers le bouchon avant 54. Ces tirants 74 sont fixés par une extrémité avant au manchon conique 36 de la pince 34, l'extrémité arrière des tirants 74 s'étendant en porte-à-faux dans le corps 22. Les extrémités arrières des tirants comprennent des moyens d'arrêt 76 en translation du coulisseau 70. En l'occurrence, les moyens d'arrêt sont formés par des anneaux élastiques 76 fixés respectivement aux l'extrémités libres des tirants 74. Le coulisseau 70 peut ainsi coulisser entre la face arrière 66 et les anneaux élastiques 76, la dimension des entretoises 64 étant prévue pour que dans sa position extrême arrière (coulisseau en butée contre les anneaux élastiques 76) le coulisseau puisse encore se déplacer vers l'arrière pour tirer le manchon de conique 36 et commander par là-même l'ouverture des mâchoires de la pince 34.

La pince 34a est montée dans un alésage traversant 78 du coulisseau 70, la partie arrière 52a faisant saillie depuis l'arrière du coulisseau 70. Le ressort 56a s'étend autour de la partie arrière 52a entre le manchon conique 36a et des moyens d'arrêt 80 ménagés à l'extrémité de la partie arrière 52a. Les moyens d'arrêt 80 peuvent être avantageusement formés par un anneau élastique. On notera que le ressort 56a est monté de manière à agir sur le manchon conique 36a afin qu'il commande la fermeture de la pince 34a. On notera également que le bouchon arrière comprend dans sa partie avant un logement 82 pouvant recevoir la partie arrière 52a de la pince 34a.

Le corps 22 comporte deux ouvertures oblongues 84 (figures 3 et 4a) qui s'étendent dans le sens longitudinal du corps 22 et qui débouchent dans l'alésage de ce corps. Le coulisseau 70 est relié par des moyens de liaison 86 qui s'étendent à travers les ouvertures 84 à des moyens premiers d'actionnement 88 et des deuxièmes moyens d'actionnement 90 capables de coulisser indépendamment les uns des autres sur la surface extérieure du corps 22.

Les premiers moyens d'actionnement 88 comprennent un manchon tubulaire 92 sur lequel est chassée une douille d'actionnement 94 de manière à former un ensemble avec un épaulement 96. Une partie extrême arrière du manchon 92 comprend deux trous radiaux diamétralement opposés dans lequel sont respectivement logés des premières goupilles 98 ou analogues, ces dernières pénétrant dans des trous de deux clavettes d'entraînement 100 logées respectivement dans des rainures 102 du coulisseau 70 prévues à cet effet. Les clavettes 100 sont à leur tour reliées en translation au manchon 92 par l'intermédiaire de deuxièmes goupilles 104 pénétrant chacune dans un trou 105 ménagé dans le fond des rainures 102. Les goupilles 98, et 104, et les clavettes d'entraînement 100 forment ainsi les moyens de liaison 86.

Les deuxièmes moyens d'actionnement 90 présentent la forme extérieure générale d'une bobine 106 comportant un rebord avant 108, une partie sensiblement cylindrique 110 et un rebord arrière 112. La bobine est traversée par un alésage comportant une partie avant 114 et une partie arrière 116. La partie avant 114 présente un diamètre supérieure au diamètre extérieur du corps 22 pour pouvoir recevoir une partie arrière du manchon 92 et former avec la surface extérieure du corps 22 et la paroi intérieure de la partie avant 114 un logement 118 pour des moyens de rappel 120. La partie arrière 116 est quant à elle ajustée à coulissement sur le diamètre extérieur du corps 22.

La partie avant 114 comporte un filetage intérieur sur lequel est vissé une virole 122 qui est logée à l'intérieur de la douille 94. La virole 122 est bloquée en rotation par rapport à la douille 94 au moyen d'une goupille d'arrêt 124 qui s'étend à la fois dans la douille et 94 et dans la virole 122 (figure 3).

Enfin une bague 126 formant butée est chassée sur l'extrémité arrière du corps 22 afin de coopérer avec le rebord arrière 112 de la bobine.

Ainsi, les moyens d'actionnement 88 agissent simultanément sur les pinces 34 et 34a pour saisir ou libérer la gaine 6 et le câble 14 tandis que les deuxièmes moyens d'actionnement 90 n'agissent que sur la pince 34a pour commander la translation relative de la gaine 6 et du câble 14 et par là-même la pince à biopsie.

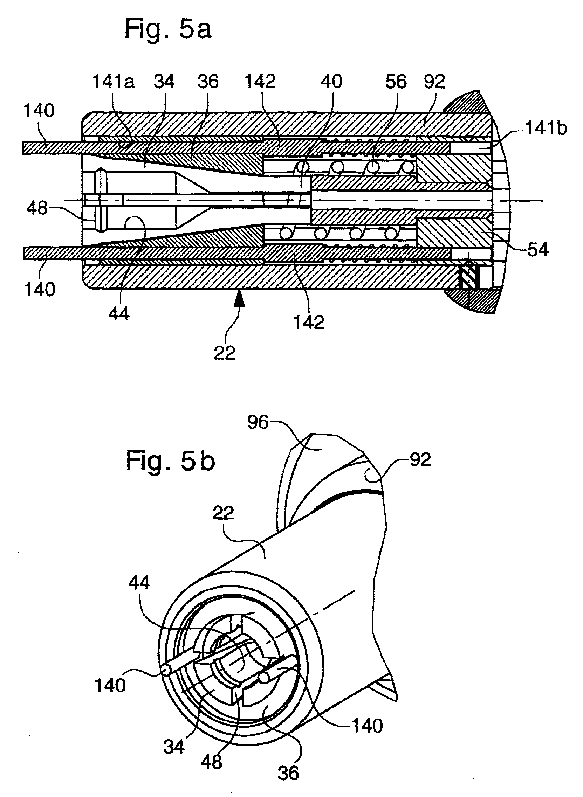

En se référant aux figures 5a et 5b, on voit une variante de réalisation de l'unité de commande 4 dans laquelle on a prévu des moyens d'éjection de l'unité fonctionnelle 2. Ces moyens d'éjection permettent, à l'aide d'une seule main et par une simple manipulation de la douille 94, d'éjecter la partie proximale de la gaine dès l'ouverture de la pince 34. Dans l'exemple illustré, ces moyens d'éjection comprennent deux poussoirs 140 qui coulissent parallèlement à l'axe longitudinal de l'unité 4 dans des passages 141a et 141b prévus dans le manchon conique 36 et dans le bouchon avant 54. Chaque poussoir 140 comprend dans sa partie médiane une portion 142 ayant une section de dimension supérieure à celle des passages 141a et 141b et s'étendant entre la face arrière 60 du manchon 36 et la face avant 58 du bouchon 54. Chaque poussoir 142 est également associé à des moyens de rappel 144 agencés pour que le poussoir 142 pousse l'extrémité proximale 8 de la gaine 6 vers l'extérieur de l'unité de commande 4. Dans l'exemple décrit les moyens de rappel 144 comprennent un ressort hélicoïdal disposé autour du poussoir, le ressort étant caler entre la face avant du bouchon et la portion 142.

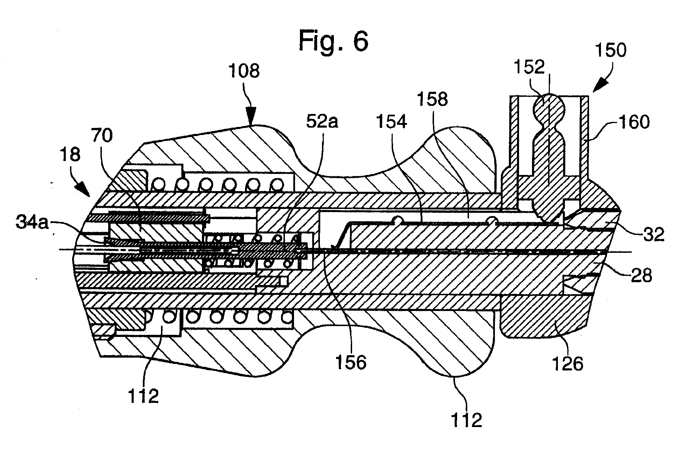

En se référant à la figure 6, on voit une autre variante réalisation de l'unité de commande 4 dans laquelle on a prévu des moyens de connexion électrique 150 permettant de porter les deuxièmes moyens de couplage 18 à un potentiel déterminé. Dans l'exemple illustré, les moyens de connexion 150 comprennent une fiche de contact 152 reliée électriquement aux moyens de couplage 18 par l'intermédiaire d'un conducteur électrique 154, ayant la forme d'une lame métallique, en contact avec une tige métallique 156 qui est fixée à l'extrémité arrière de la pince 34a des moyens de couplage 18. La fiche de contact 152 est prévue dans une partie fixe de la région proximale de l'unité de commande 2 et plus précisément dans la bague 126 qui se trouve à l'extrémité arrière de l'unité de commande 4. On notera que dans cette variante le bouchon arrière 28 est prolongé et fait saillie depuis l'arrière de l'unité 4 et comprend une rainure longitudinale 158 dans laquelle s'étend la lame 154. La bague 126 est montée sur la partie faisant saillie du bouchon 28. La bague 126 comprend une douille cylindrique 160 dont l'axe s'étend perpendiculairement à l'axe longitudinal de l'unité 4, et dont l'ouverture débouche dans la rainure 158. La fiche de contact 152 est chassée dans la douille et vient directement en contact avec la lame 154. Il va de soi que dans cette variante, le bouchon arrière 28, le coulisseau 70 et les clavettes 100 seront réalisées en un matériau isolant ou le cas échéant revêtus d'un gainage isolant.

Le fonctionnement de l'instrument chirurgical selon l'invention va maintenant être décrit en détail ci-après. On décrira successivement (A) la mise en place de l'unité fonctionnelle 2 sur l'unité de commande 4, (B) la manoeuvre de l'instrument 1 et (C) la séparation de l'unité fonctionnelle 2 de l'unité de commande.

### A. Mise en place

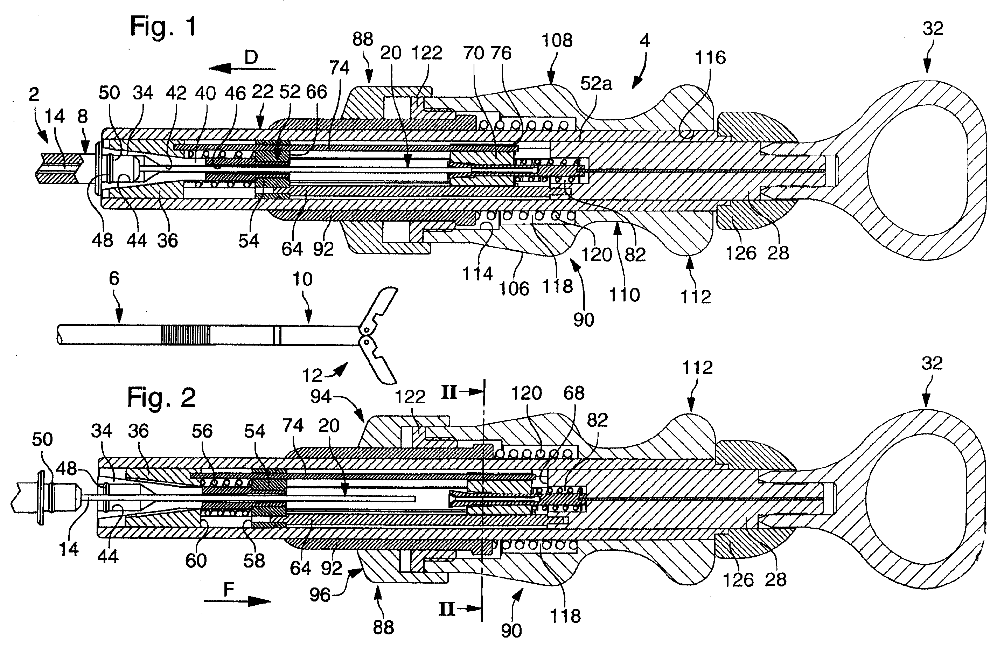

On procède tout d'abord à l'ouverture des pinces 34 et 34a, par exemple en agissant avec l'index et le majeur sur l'épaulement 96 de la douille 94 de manière à la déplacer vers l'arrière (dans direction de la flèche F, figure 2) tout en maintenant la poignée 32 avec le pouce de la même main. La bobine vient alors en butée par son rebord arrière 112 contre la bague 126. au cours de celle opération, La douille 94 entraîne également l'équipage mobile 62 dans la même direction F par l'intermédiaire des clavettes d'entraînement 100 et des goupilles 98 et 104. Le coulisseau 70 vient alors en butée contre les anneaux élastiques 76 fixés sur les tirants 74. On continue alors de déplacer la douille 94 dans la direction F. Ce faisant, le coulisseau 70 déplace par l'intermédiaire des tirants 74 le manchon conique 36 dans la direction F ce qui commande l'ouverture de la pince 34. Simultanément, la partie arrière 40a de la pince 32a vient butée contre le fond du logement 82 ce qui dégage le manchon conique 36a de la partie avant de la pince 34a et commande son ouverture. A la fin de cette opération les ressorts 56, 56a et 120 sont comprimés et les mâchoires des pinces 34 et 34a sont ouvertes. L'extrémité proximale 8 de la gaine 6 et l'extrémité proximale 20 du câble 14 peuvent être alors respectivement introduites dans les pinces 34 et 34a. On relâche ensuite la douille 94. Le ressort 120 rappelle alors la douille 94 dans une direction D opposée à la direction F afin d'écarter la douille 94 de la bobine 106. Simultanément, les ressorts 56 et 56a agissent respectivement sur les manchons 36 et 36a pour les déplacer dans la direction D et commander ainsi la fermeture des pinces 34 et 34a. L'unité fonctionnelle 2 est alors attachée à l'unité de commande 4 (figure 1).

### B. Manoeuvre de l'instrument

La manoeuvre de l'instrument chirurgical selon l'invention s'effectue en agissant sur l'unité de commande 4. Plus précisément, elle s'effectue en agissant sur la bobine 106 pour la faire coulisser le long des directions F et D. Les pinces 34 et 34a sont fermées, et la bobine 106 actionne le câble 14, par l'intermédiaire de la clavette d'entraînement 100 et des goupilles 98 et 104, pour le déplacer relativement à la gaine 6. Bien entendu, les mâchoires de la pince 34a sont maintenues fermées sous l'effet du ressort 56a qui agit sur le manchon conique 36a pendant cette opération. Le coulisseau 70 coulisse librement sur les entretoises 64 et les tirants 74, les pinces 34 et 34a étant maintenues fermées pendant le mouvement de la bobine 106.

### C. Opération de séparation

L'opération de séparation de s'effectue en commandant l'ouverture des pinces 34 et 34a par action sur la douille 94 comme cela a été décrit en liaison avec l'opération A.

Bien entendu dans le cas où l'unité de commande 4 comprend des moyens d'éjection 110, les poussoirs 112 éjectent automatiquement la gaine 6 et le câble 14 dès l'ouverture des pinces 34 et 34a.

## Revendications

1. Instrument chirurgical comprenant:
- une unité fonctionnelle et une unité de commande pouvant être séparée de celle-ci, l'unité fonctionnelle comprenant une gaine ayant une extrémité proximale et une extrémité distale, un outil monté à l'extrémité distale de la gaine, et un câble coulissant à l'intérieur de la gaine reliant ledit outil à l'unité de commande, et permettant d'actionner l'outil lorsque l'on agit sur ledit câble au moyen de l'unité de commande,
- l'unité de commande comprenant des premiers et des deuxièmes moyens de couplage pour relier, de façon amovible, l'unité de commande respectivement à l'extrémité proximale de la gaine et à l'extrémité proximale du câble caractérisé en ce que
l'unité de commande comprend des premiers et deuxièmes moyens d'actionnement, et en ce que les premiers moyens d'actionnement agissent simultanément sur les premiers et deuxièmes moyens de couplage pour saisir ou libérer ladite gaine et ledit câble tandis que les deuxièmes moyens d'actionnement n'agissent que sur les deuxièmes moyens de couplage pour commander la translation relative de la gaine et du câble.

2. Instrument chirurgical selon la revendication 1, caractérisé en ce que lesdits premiers et deuxièmes moyens de couplage comprennent respectivement des premier et deuxième organes de saisie mobiles entre une première position dite d'ouverture dans laquelle les extrémités proximales respectives de la gaine et du câble peuvent être introduites et une deuxième position dite de fermeture dans laquelle les extrémités proximales respectives de la gaine et du câble peuvent être maintenues fixement.

3. Instrument chirurgical selon la revendication 2, caractérisé en ce qu'il comprend des premiers et deuxièmes moyens de rappel respectivement des premiers et deuxièmes moyens de couplage dans leur position de fermeture respective.

4. Instrument chirurgical selon la revendication 1, caractérisé en ce que les premiers moyens d'actionnement coopèrent avec un équipage mobile portant les deuxièmes moyens de couplage, et en ce que ledit équipage mobile est relié audits deuxièmes moyens de couplage.

5. Instrument chirurgical selon la revendication 4, caractérisé en ce que l'équipage mobile est relié audits deuxièmes moyens de couplage par un tirant sur lequel il peut coulisser.

6. Instrument chirurgical selon la revendication 4, caractérisé en ce que ledit tirant amène les premiers moyens de couplage dans la position d'ouverture lorsque l'on agit sur lesdits premiers moyens d'actionnement à l'encontre desdits premiers moyens de rappel .

7. Instrument chirurgical selon l'une des revendications 1 à 6, caractérisé en ce que les premiers et deuxièmes moyens de couplage sont disposés dans un corps allongé creux sur lequel coulissent lesdits premiers et deuxièmes moyens d'actionnement.

8. Instrument chirurgical selon la revendication 7, caractérisé en ce que ledit corps comporte une ouverture oblongue à travers laquelle s'étendent des moyens de liaison reliant lesdits premiers moyens d'actionnement audit équipage mobile.

9. Instrument chirurgical selon la revendication 8, caractérisé en ce que lesdits premiers moyens d'actionnement coulissent par rapport audit corps et par rapport audits deuxièmes moyens d'actionnement et en ce que lesdits deuxièmes moyens d'actionnement coopèrent une butée solidaire du corps.

10. Instrument chirurgical selon la revendication 9, caractérisé en ce que des troisièmes moyens de rappel sont prévus entre les premiers et les deuxièmes moyens d'actionnement pour écarter les premiers et les deuxièmes moyens d'actionnement les uns des autres.

11. Instrument chirurgical selon l'une des revendications précédentes caractérisé en ce que les premiers et deuxièmes moyens de couplage comprennent respectivement un premier et un deuxième dispositifs à pince.

12. Instrument chirurgical selon la revendication 11, caractérisé en ce que lesdits premier et deuxième dispositifs à pince comprennent une première et une deuxième pinces à ouverture et fermeture associés respectivement à un premier et un deuxième manchon de commande conique.

13. Instrument chirurgical selon la revendication 12, caractérisé en ce que lesdits premiers et deuxièmes moyens de rappel s'étendent respectivement entre les premier et deuxième manchons de commande et des première et deuxième surfaces de butée.

14. Instrument chirurgical selon la revendication 13, caractérisé en ce que les première et deuxième surfaces de butée sont respectivement formées par une face d'un premier et d'un deuxième bouchon montés dans ledit corps, ledit premier bouchon étant disposé entre la première et la deuxième pince, et traversé par ledit tirant.

15. Instrument chirurgical selon la revendication 14, caractérisé en ce qu'une poignée de manipulation est fixée à une extrémité dudit deuxième bouchon.

16. Instrument chirurgical selon l'une des revendications précédentes, caractérisé en ce que l'unité de commande comprend en outre des moyens d'éjection de l'unité fonctionnelle.

17. Instrument chirurgical selon la revendication 16, caractérisé en ce que les moyens d'éjection comprennent au moins un poussoir qui coulisse dans la partie distale de l'unité de commande, et qui est associé à des moyens de rappel agencés pour que le poussoir pousse l'extrémité proximale de l'unité fonctionnelle vers l'extérieur de l'unité de commande.

18. Instrument chirurgical selon l'une des revendications précédentes, caractérisé en ce que l'unité de commande comprend en outre des moyens de connexion électrique permettant de porter les deuxièmes moyens de couplage à un potentiel déterminé.

19. Instrument chirurgical selon la revendication 18, caractérisé en ce que les moyens de connexion électrique comprennent une fiche de contact reliée électriquement au deuxième moyens de couplage au moyen d'un conducteur électrique.

20. Instrument chirurgical selon la revendication 19 et la revendication 18, caractérisé en ce que la fiche de contact est prévue dans une partie fixe de la région proximale de l'unité de commande.
